# EUROPEAN PATENT APPLICATION

(11) **EP 4 592 380 A2**
(43) Date of publication of application: **30.07.2025**
(21) Application number: 25163162.8
(22) Date of filing: 12.03.2025
(51) Int. Cl.: C12N 5/00

(54) **CONTINUOUS HANDLING OF SUSPENSION CELL CULTURES**

(71) Applicant: Bayer Aktiengesellschaft, 51373 Leverkusen (DE)
(72) Inventor: Sousa, Marcos, 51373 Leverkusen (DE); Krasenbrink, Jürgen, 51373 Leverkusen (DE); Poggel, Martin, 51373 Leverkusen (DE); Teale, Misha, 8400 Winterthur (CH); Schneider, Samuel, 8400 Winterthur (CH); Eibl-Schindler, Regine, 8400 Winterthur (CH)
(74) Representative: BIP Patents

(57) **Abstract**

What is described herein relates to a method for continuous handling of cells in a suspension cell culture, wherein the suspension cell culture is provided in a vessel of a size in the range of 0,2l - 100l and the continuous in-situ handling comprises washing and/or dissociating and/or harvesting the cells without pausing agitation.

## Description

### FIELD OF THE INVENTION

The disclosure relates to novel methods for continuous handling of suspension cell cultures.

### BACKGROUND

Suspension cell culture is a well-known technique for expanding cell populations, such as stem cells as well as various further e.g. also terminally differentiated cell types. However, while there is vast knowledge available on performing suspension culture on research scale, larger scale cell culture is needed, for example, for process development, commercial production of cells, cell expansion for clinical trials and/or cell banking. Especially for these larger cell culture scales, cost and time efficient methods for handling of the cells are required which are operator independent and minimize contamination risks.

### DETAILED DESCRIPTION

What is described herein relates to a method for continuous handling of cells in a suspension cell culture, wherein the suspension cell culture is provided in a vessel of a size in the range of 0,2 1 - 100l and the continuous handling comprises washing and/or dissociating and/or harvesting the cells without pausing agitation.

In the state of the art filters with varying pores sizes are disclosed to remove cell culture medium by suctioning from the culture solution while the cultured cells remain in the culture vessel hence enabling perfusion cell culture (cf. e.g. US20240101966A for microcarriers or Roldão et al. 2018 Culture of 3D Cell Aggregates in Perfusion in a DASbox® Mini Bioreactor System, Eppendorf Application Note No. 360 I March 2018 for cell aggregates). Moreover, WO2024008773A1 describes that at the day of harvest, cells were dissociated in the same vessel that was used for culturing via aspiration of most of the medium with a dip tube of fixed height after pausing the agitation in order for the cells to settle prior to medium aspiration. In addition, Schneider et al. 2025 describe that microcarrier retention during suspension cell culture was ensured by a custom-built mesh covered dip tube (pore diameter ≈ 70-80 µm), which was also used during harvest operations.

The method described herein compared to the methods of the state of the art allows for continuous handling of the cells in-situ i.e. washing and/or dissociating and/or harvesting the cells without pausing agitation. This has the advantage that the optimal culture conditions supplied evenly through the cell culture vessel via agitation are not interrupted but continuously present.

In an embodiment the agitation during washing and/or dissociating and/or harvesting of the cells is carried out at a stirrer rate in the range of 5-1250 rpm, preferably 5-570 rpm, most preferably 5-170 rpm and/or a *P*/*V_{W}* (power input per working volume) of 0.1-336 W m⁻³ , preferably 0.1- 152 W m⁻³ , most preferably 0.1- 77 W m⁻³. The skilled person is aware that the chosen rpm value depends on the vessel size and cell culture volume whereas the power input per working volume is more depending on factors such as the used cell type and the cell culture stage i.e. expansion, differentiation etc.. In a preferred embodiment the agitation during the washing and/or dissociating and/or harvesting of the cells is carried out at a stirrer rate in the range of 40-60 rpm and/or a *P*/*V_{W}* (power input per working volume) of 0.37-0.74 W m⁻³.

In another preferred embodiment the washing of the cells is carried out with a volume which is in the range of 0 liters to 3 times the cell culture volume.

In another preferred embodiment the washing of the cells is carried out with a volume which is in the range of 0 liters to 100 times the cell culture volume.

In another embodiment the cells are cultured on microcarriers of a size in the range of 60µm - 250µm. To a skilled person it is clear that the microcarrier choice depends on the used cell type and the used cell culture vessel. Various types of microcarriers with or without coatings a commercially available including alginate-based (GEM, Global Cell Solutions), dextran-based (Cytodex, GE Healthcare), collagen-based (Cultispher, Percell), and polystyrene-based (SoloHill Engineering) microcarriers varying in size between 60-380µm (cf. for example Table 1 of Li B, Wang X, Wang Y, Gou W, Yuan X, Peng J, et al. (April 2015). "Past, present, and future of microcarrier-based tissue engineering". Journal of Orthopaedic Translation. 3 (2): 51-57).

In a preferred embodiment the cells are cultured on microcarriers of a size in the range of 100 µm - 250µm.

In another embodiment washing and/or dissociating and/or harvesting of the cells without pausing agitation occurs via a dip tube comprising a filter with a pore size in the range of 30µm -200 µm. To a skilled person it is clear that the pore size depends on the size of the used microcarrier i.e. the smaller the used microcarrier the smaller the pore size of the filter. Hence in a preferred embodiment washing and/or dissociating and/or harvesting of the cells without pausing agitation occurs via a dip tube comprising a filter with a pore size in the range of 60-80µm.

### EXPERIMENTAL SECTION

Between 1 - 4 × 10⁵ cells mL⁻¹ iPS cells were seeded into BioBlu 1c bioreactors (Eppendorf) and allowed to attach to Synthemax II low concentration polystyrene microcarriers (SynP MCs). After attachment the cells were cultured in perfusion mode with a maximum *P*/*V_{W}* (power input per working volume) of 0.37-0.74 W m⁻³ and *N* of around 57 rpm in 1,31 cell culture medium. To allow for perfusion mode a triple-port with two ports occupied by level probes and the final position occupied by a microcarrier retention probe was attached to the BioBlu 1c bioreactors. The height of the level probes was adjusted so that the anti-foam pump was not activated once the maximum *V_{W}* was reached. Rather than anti-foam solution, the anti-foam pump connected the waste to the microcarrier retention (MC) probe. Furthermore, the tip of the MC retention probe was specifically designed with a pore size of between 70-80 µm, meaning, that once the maximum *V_{W}* was reached and spent medium removal began, retention of the SynP MCs, which had a minimum diameter of 125 µm, was assured. The level probes ensured that a constant volume was kept in the bioreactor via adjusting the pump flow adding fresh medium according to the constant flow that continuously removed medium from the bioreactors. Cell harvest from the BioBlu 1c bioreactors was performed under dynamic conditions. Briefly, the spent medium was removed at an agitation of 57 rpm from the BioBlu 1c while retaining the microcarriers. The cells were washed with 0 to 4L of PBS before a detachment agent was added. Following the detachment phase the detachment agent was quenched via adding cell culture medium. Finally, the cells were harvested. During the whole harvest procedure agitation here stirring continued with at least 57 rpm. Cells were replated to observe conservation of attachment ability post-harvest, as well as trilineage potency. Cell pluripotency and trilineage potency were maintained in all instances. Cell densities were higher than in adherent cell cultures established in parallel with the BioBlu 1c cell culture.

## Claims

1. Method for continuous handling of cells in a suspension cell culture, wherein the suspension cell culture is provided in a vessel of a size in the range of 0,2l - 100l and the continuous handling comprises washing and/or dissociating and/or harvesting the cells without pausing agitation.

2. Method according to claim 1 wherein the agitation during washing and/or dissociating and/or harvesting the cells is carried out at a stirrer rate in the range of 40-60 rpm and/or a power input per working volume *P*/*V_{W}* of 0.37-0.74 W m⁻³.

3. Method according to claim 1 or claim 2 wherein the cells are cultured on microcarriers of a size in the range of 100µm - 250µm.

4. Method according to claim 1 wherein washing and/or dissociating and/or harvesting the cells without pausing agitation occurs via a dip tube comprising a filter with a pore size in the range of 60- 80µm.
